# EUROPEAN PATENT APPLICATION

(11) **EP 4 293 010 A1**
(43) Date of publication of application: **20.12.2023**
(21) Application number: 22179164.3
(22) Date of filing: 15.06.2022
(51) Int. Cl.: C07C 67/03, C07C 67/54, C07C 69/54

(54) **PROCESS FOR THE DISCONTINUOUS (TRANS)ESTERIFICATION OF (METH)ACRYLATE COMPOUNDS**

(71) Applicant: Evonik Operations GmbH, 45128 Essen (DE)
(72) Inventor: SOLER, Nicolas, 63743 Aschaffenberg (DE); FINKLER, Tiago Fiorenzano, 63150 Heusenstamm (DE); KEHR, Thomas, 64367 Mühltal (DE); LUFT, Heiko, 63110 Rodgau (DE); MAUL, Christian, 67435 Neustadt a. d. W. (DE)
(74) Representative: Evonik Patent Association

(57) **Abstract**

The present invention discloses process strategies and a method for the discontinuous (trans)esterification of (meth)acrylic esters. The method can prepare an alkyl (meth)acrylate product by a (trans)esterification reaction of a reaction mixture. The reaction mixture comprises a (meth)acrylate starting material and a first alcohol which are converted by the (trans)esterification reaction in the presence of a catalyst into the alkyl (meth)acrylate product and a side product. The (trans)esterification reaction is carried out in a reactor system. The reactor system comprises a heating and/or cooling system, a reaction chamber comprising the reaction mixture, a column with a column head, a vapor transfer line, a condenser, a reflux tank, a reflux line, a distillate transfer line, and a receiver vessel. over a predominant amount of time during the (trans)esterification reaction at least a portion of the side product is continuously removed by distillate take off. The pressure is constantly adjusted to keep the reaction temperature below a predetermined upper limit.

## Description

### Field of the invention

The invention relates to the field of discontinuous (trans)esterification of (meth)acrylate compounds with an alcohol to produce a target product accompanied by the separation of a side product.

### Background of the invention

For the discontinuous (trans)esterification of (meth)acrylic esters with an alcohol, amino alcohol or amine to produce a new target product, a heatable reactor is used which has a directly connected column as a separation system. The reactor can be evacuated via a vacuum system. The vacuum can be regulated as required. The pressure in the reactor system can be chosen freely. This means that an overpressure in the reactor and column and condensation system is also possible. The vapors from the column are condensed and a partial or the full stream is discharged into a receiver vessel. The remaining condensate is used as a return flow for the columns and is returned to the reaction system via the column sump.

In the conventional mode of operation, more of the required (meth)acrylic esters (e.g. MMA - methyl methacrylate) than alcohol, amino alcohol or amine has to be present before starting the reaction with the alcohol. The reason for this is that the required (meth)acrylic esters are not only used as a starting material for the targeted product to be produced but also as a boiling aid for limiting the temperature of the reaction, as in the course of the reaction, the composition of the reaction mixture changes due to product formation, so that the presence of a further high-boiling component increases the boiling point of the mixture and thus also the overall reactor temperature. Limiting the temperature of the reaction is important to prevent unintentional polymerization of the (meth)acrylic esters and the target product. By providing the excess of (meth)acrylic esters, the reactor volume cannot be fully used for the raw materials that are to generate the target product, which leads to a limited space-time yield. Likewise, larger quantities of required (meth)acrylic esters must be recycled or are disposed of as excess, which costs time, energy, and money.

Therefore, the objective of the present invention is to provide a method that overcomes the aforementioned problems.

### Summary of the invention

The invention is directed to a method for preparing an alkyl (meth)acrylate product by a (trans)esterification reaction of a reaction mixture in a reactor system, the reactor system comprising a heating and/or cooling system, a reaction chamber comprising the reaction mixture, a column with a column head, one or more vapor transfer lines, a (multistage) condenser system, a reflux tank, a reflux line, a distillate transfer line, and a receiver vessel,
wherein the reaction mixture comprises a (meth)acrylate starting material and a first alcohol, amino alcohol or amine which are converted by the (trans)esterification reaction at a reaction temperature and a given pressure in the reaction chamber in the presence of a catalyst into the alkyl or amino (meth)acrylate product and a side product,
wherein over a predominant amount of time during the (trans)esterification reaction at least a portion of the side product is continuously removed by distillate take off, and
wherein over a predominant amount of time during the (trans)esterification reaction the given pressure is constantly adjusted to keep the reaction temperature below a predetermined upper limit.

To prevent the reaction from exceeding a temperature threshold in the reactor without the temperature stabilizing effect of (meth)acrylic esters, which would prevent polymerization, the pressure in the system is constantly adjusted so that the reaction temperature is kept below a predetermined upper limit. In this process, the pressure is lowered linearly to a target value as the temperature naturally increases in the reactor as the reaction process proceeds. This not only has a positive effect on the reduced polymerization tendency, but also the average reaction rate achieved over the batch time is positively influenced.

### Description of the drawings

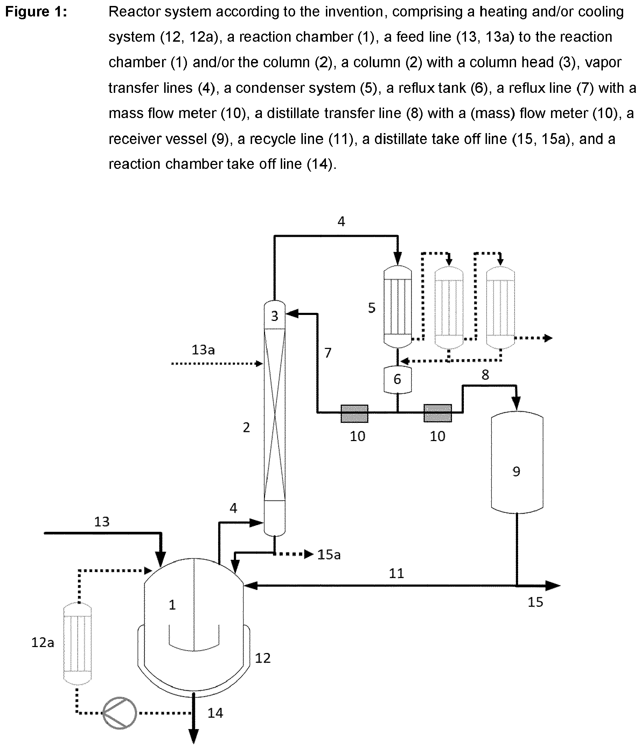

**Figure 1** illustrates a reactor system according to the invention, the reactor system comprises a heating and/or cooling system (12, 12a), a reaction chamber (1), a feed line (13, 13a) to the reaction chamber (1), a column (2) with a column head (3), a vapor transfer line (4), a (multistage) condenser system (5), a reflux tank (6), a reflux line (7) with a mass flow meter (10), a distillate transfer line (8) with a mass flow meter (10), a receiver vessel (9), a recycle line (11), a distillate take off line (15, 15a), and a reaction chamber take off line (14).

### Detailed description of the invention

Unless otherwise particularly defined herein, the related terms used in the present invention have the following definitions.

As used herein, the term "(meth)acrylate" refers to both (meth)acrylic acid and (meth)acrylic acid ester. Furthermore, it refers to both methacrylate and acrylate. Or it refers to methacrylate or acrylate depending on the respective context. It also refers to methacrylic acid and methacrylic acid ester. For example, the term "a (meth)acrylate compound" or "a (meth)acrylate product" refers to (meth)acrylic acid and a (meth)acrylic acid ester, e.g. an alkyl (meth)acrylate.

Preferred (meth)acrylate products that can be prepared according to the present application are selected from the group consisting of:
dimethylaminoethyl methacrylate
1,4-butanediol dimethacrylate
ethyl methacrylate
dimethylaminopropyl methacrylate
benzyl methacrylate
allyl methacrylate
2-ethylhexyl methacrylate
ethyleneglycol dimethacrylate
butyldiglycol methacrylate
triethyleneglycol dimethacrylate
1,3-butanediol dimethacrylat
trimethylolpropane trimethacrylate
ethyltriglycol methacrylate
1,6-hexanediol dimethacrylate
polyethyleneglycol 200 dimethacrylate (molecular weight = 200 g/mol)
methoxy-polyethyleneglycol methacrylate (MPEG500, molecular weight = 500 g/mol)
methacrylic acid ester having an average number of 17.4 carbon atoms
isodecyl methacrylate
methacrylic acid ester having an average number of 13 carbon atoms (synthetic lauryl methacrylate)
n-hexyl methacrylate
methacrylic acid esters of mixtures of ethoxylated C16-18 fatty alcohols

As catalyst can be used lithium chloride, lithium amide, lithium hydroxide, lithium methanolate, zirconium(IV) acetylacetonate, titanates like tetra-isopropylorthotitanate, calcium hydroxide, and calcium oxide and a combination of the aforementioned.

As used herein, the term "(trans)esterification" or "(trans)esterification reaction" refers to both an esterification reaction and a transesterification reaction. Or it refers to either an esterification reaction or a transesterification reaction depending on the respective context.

As used herein, the term "reactor system" refers to a reactor in which a chemical reaction of a reaction mixture can take place, and wherein the contents of the reactor can be subjected to a distillation process before the course of a chemical reaction and/or during the course of a chemical reaction and/or after the course of a chemical reaction or independently of a chemical reaction. The advantage of such a reactor is that it is not necessary to provide several chambers, one for a reaction to take place and one for the substance or substances to be distilled, thus saving material, time for a transfer of the substances and costs. The reactor system comprises at least an internal and/or external heating and/or cooling system, a reaction chamber, a column with a column head, a feed line to the reaction chamber or to the column, a vapor transfer line, a (multistage) condenser system, a reflux tank, a reflux line, a distillate transfer line, and a receiver vessel.

As used herein, the term "starting material" refers to raw material, initial material, educts, feedstock, reactants or initial reactants which can be used to undergo a chemical reaction, whereby the chemical reaction may result at least in a product or products, which can include side-products or by-products.

As used herein, the term "heating and or cooling system" refers to an apparatus capable of heating and, if required, of cooling a liquid and/or converting a liquid to its vapor or, in other words, gaseous state.

As used herein, the term "reaction chamber" refers to a container in which a chemical reaction is carried out. There are a wide variety of chambers being referred to as such. For example, the sizes of reaction chambers range from micro reaction chambers, which hold a few microliters, to reaction chambers for a few milliliters, to chambers with a volume of numerous cubic meters. The most important characteristic of each reaction chamber is its resistance to the reaction conditions.

As used herein, the term "reaction mixture" refers to a mixture of substances that participate in a reaction. Such a reaction mixture can comprise or consist of
- the starting material(s),
- the starting material(s) and possible additive(s) such as auxiliary substances, which can be, for example, catalysts or other reaction accelerators, and/or stabilizer(s),
- the starting material(s) and product(s) including possible side-product(s),
- the starting material(s), additive(s) and product(s) including possible side-product(s),
- the product(s) including possible side-product(s) and the additive(s),
- the product(s) alone and additive(s),
- the product(s) including possible side-product(s), or
- the product(s) alone.

As used herein, the term "column" refers to an apparatus for the thermal separation of mixtures. To avoid heat loss, the column can be an insulated, preferably cylindrical, tube, which can be made in particular of steel, high-alloy stainless steels, glass or plastic. The height of the column body can mainly be dictated by the required quality of separation; the diameter by the volume flow of the mixture to be separated. The column can be placed between the reaction chamber and the distillation head. The number of individual distillations required for the same separation performance can also be referred to as the "theoretical plate number". At the surface of the column, the equilibrium between the liquid and gaseous phases can constantly be re-established by condensation and evaporation. As a result, the proportion of the low-boiling component continues to increase towards the top, while the higher-boiling component flows back into the reaction chamber, the sump. The size of the surface area of the column can be greatly increased in various ways by the design of trays, as in the Vigreux column, or by filling with packing or structured packing.

As used herein, the term "feed line" refers to supply lines which feed substances or substance mixtures to e.g. the reaction chamber or to the column.

As used herein, the term "condenser system" refers to a unit in which the vapor produced during distillation, which can be composed of the various volatile components of the solution to be separated, can liquefy by cooling through one or more different cooling liquids.

As used herein, the term "reflux tank" refers to a container into which condensed distillate flows, which then either flows back to the column and/or into the reaction chamber or is removed from the system.

As used herein, the term "reflux line" refers to a line which can feed distillate from the reflux tank back to the column or to the reaction chamber.

As used herein, the term "distillate transfer line" refers to a line which can remove at least a portion of distillate from the reflux tank, and, thus, deprive distillate from the reflux to the column or column head.

The term "distillate take off line" refers to a line which can remove at least a portion of distillate in general from the reactor system and the distillation system.

As used herein, the term "starting material" refers to raw material, initial material, educts, feedstock, reactants or initial reactants which can be used to undergo a chemical reaction, whereby the chemical reaction may result at least in a product or products, which can include side-products or by-products.

As herein the term "reaction time" is defined as the duration in which the side product is removed from the reaction mixture. The reaction time strongly depends on the first alcohol used in the transesterification reaction and can vary between 2 hours and up to 48 hours.

As used herein, the term "predominant amount of time" means more than 50% of the reaction time, more preferred than 60% and most preferred more than 75% of the reaction time.

As used herein, the term "target value" refers to a value to be reached within a process. In particular, a target value refers to a specified pressure that is to be reached in the course of the (trans)esterification reaction.

As used herein, the term "column head concentration" refers to a concentration of distillate in the column head. The distillate can be any compound of the reaction mixture or its side-products or by-products.

As used herein, the term "vacuum phase" refers to a phase of the product generation, where low pressure or vacuum is applied to the reactor.

The term "distillation phase" refers to a phase of the product generation in which substances of the reaction mixture are distilled of.

As used herein, the term "high-boiling esters" or "high-boiling component" refers to esters having a boiling point of 210°C or above, for example, ethylene glycol dimethylacrylate (EGDMA), methacrylates having an average number of 13 C-atoms, 1,3-butanediol dimethacrylate (1,3 BDDMA), and 1,4-butanediol dimethacrylate (1,4 BDDMA).

The term "low-boiling esters" or "low-boiling component" refers to esters having a boiling point of below 210 C, for example 2-ethylhexyl methacrylate (EHMA), isodecyl methacrylate (IDMA), and butyl methacrylate (BUMA).

As used herein, the term "wt%" refers to weight percentage.

The problem underlying the present invention is solved by a method for preparing an alkyl (meth)acrylate product by a (trans)esterification reaction of a reaction mixture in a reactor system, the reactor system comprising a heating and/or cooling system, a reaction chamber comprising the reaction mixture, a column with a column head, a vapor transfer line, a condenser system, a reflux tank, a reflux line, a distillate transfer line, and a receiver vessel,
wherein the reaction mixture comprises a (meth)acrylate starting material and a first alcohol which are converted by the (trans)esterification reaction at a reaction temperature and a given pressure in the reaction chamber in the presence of a catalyst into the alkyl (meth)acrylate product and a side product,
wherein over a predominant amount of time during the (trans)esterification reaction at least a portion of the side product is continuously removed by distillate take off, and
wherein over a predominant amount of time during the (trans)esterification reaction the given pressure is constantly adjusted to keep the reaction temperature below a predetermined upper limit.

The method according to the invention has the advantage the polymerization tendency is reduced, and the average reaction rate achieved over the batch time is positively influenced.

The reaction mixture may comprise two or more starting materials and two or more products. Further preferably, the two or more starting materials are a first educt, a second educt, and, optionally, one or more further compounds, and wherein the two or more products are a first product, a second product, and, optionally, one or more further products. The reaction mixture can further comprise additives such as reaction accelerators, reaction inhibitors, buffer, solvents, stabilizer, water, viscosity index improvers, thickeners, antioxidants, corrosion inhibitors, dispersants, high pressure additives, defoamers, catalysts or enzymes. Furthermore, side products can be formed by the reaction, which can also be present in the reaction mixture.

According to the present invention, before starting the reaction more alcohol (in mole) than (meth)acrylic esters can present in the reaction mixture. At the beginning, the ratio of the alcohol to the (meth)acrylic esters can be in the range of 1.2:0.8 to 1.0:1.0, preferably in the range of 1.2:0.8 to 1.1:0.9, or in the range of 1.2:0.8 to 1.0:1.0. The ratio refers to the hydroxyl groups present in the alcohol. While the reaction is running, additional amounts of (meth)acrylic esters can be added to the reaction mixture. During the course of the reaction, the ratio of the alcohol to the (meth)acrylic esters can decrease to a range of 1.0:1.0 to 0.7:1.3, preferably in the range of 1.0:1.0 to 0.8:1.2. The pressure in the system is constantly or incrementally adjusted to keep the reaction temperature below a predetermined upper limit. By adding more alcohol than of (meth)acrylic esters, more boiler volume can be used for product production and the space-time yield of the target product can be increased.

If the reaction was run conventionally, a temperature threshold would be exceeded in the reactor before the end of the reaction as the reaction temperature would not be sufficiently regulated by (meth)acrylate starting material as more first alcohol would be present, which would prevent polymerization. The adjustment of pressure through applying vacuum, or rather low pressure, however, prevents this process.

Preferably, the constant adjustment of the given pressure is a constant linear decrease of the given pressure to a target value.

This has the advantage that the pressure is reduced in a controlled way and a target pressure is reached in a predetermined time frame.

During the (trans)esterification reaction, the target value for the reaction temperature is in the range of 90°C to 145°C, preferably in the range of 100°C to 135°C, at a pressure in the range of 1300 mbar to 400 mbar (abs.), preferably in the range of 1150 mbar to 600 mbar (abs.). At the end of the reaction phase, the pressure may be reduced below 600 mbar to 20 mbar (abs.).

The reactor system may further comprises one or more elements selected from the group of one or more mass flow meters, a recycle line, a distillate take off line, a reaction chamber take off line, and a feed line to the reaction chamber or to the column, preferably wherein the feed line is a first feed line, and wherein the reactor system further comprises a second feed line to the reaction chamber or to the column, and, optionally, further feed lines to the reaction chamber or to the column.

This has the advantage that substances and/or mixtures can be added to the reaction chamber and/or column via several feed lines. Substances and/or mixtures can be added simultaneously or successively and/or continuously or discontinuously via these feed lines. Different substances and/or mixtures can be added to the reaction chamber and/or column via the feed lines. Also, identical substances and/or mixtures can be added to the reaction chamber and/or column via the feed lines. Furthermore, identical substances and/or mixtures and different substances and/or mixtures can be added to the reaction chamber and/or column via the feed lines. These substances or mixtures can be, for example, the starting materials used for the reaction and/or additives such as reaction accelerators, reaction inhibitors, buffer, solvents, stabilizer, water, viscosity index improvers, thickeners, antioxidants, corrosion inhibitors, dispersants, high pressure additives, defoamers, catalysts or enzymes.

Preferably, the reactor comprises a possibility to supply energy through heating and remove energy through cooling. Preferably, the reactor comprises mixing equipment for the reaction chamber. Preferably, the reactor comprises dosing means for solids, liquids, and gases, which can be provided to the reaction chamber, column, vapor lines, the condensation system and feed lines.

The reactor comprises a pressure control system which is able to control and adjust the pressure in the reactor chamber, the column and the condensation system.

Through the reflux line distillate can flow back onto the column or into the reaction chamber. The reflux tank stores reflux of the distillate.

The first alcohol can be a linear or branched C2-C20 alkanol, a linear or branched C2-C20 alkandiol, an amino alcohol, preferably dimethylaminoethanol and dimethyl aminopropanol, an amine a linear or branched vicinal C2-C20 alkandiol, or, preferably, ethylene glycol.

In the inventive method, the side product can also be removed in form of a mixture of the side product and the (meth)acrylate starting material.

This has the advantage that, especially at the end of the reaction, the reaction continues to run in the direction of product generation and does not come to a standstill.

When performing the method according to the invention, it is a preferred option that a filling level of the reaction chamber is kept constant by compensating at least a portion of the withdrawn (meth)acrylate starting material and/or of the first alcohol by adding a further amount of the (meth)acrylate starting material and/or of the first alcohol to the reaction mixture in the reaction chamber, preferably via a feed line to the reaction chamber or to the column.

The filling level of the reaction chamber is kept constant by using an inflow control, e.g. for the MMA and/or the alcohol. The feed of educts is regulated (e.g. control valve and mass flow measurement). During the course of the reaction, by-product (e.g. methanol) is withdrawn from the rection chamber via the column. The rection chamber filling level thus drops continuously if no additional educt is fed in. The filling level control now controls and regulates the feed of the educt(s). The output signal from the filling level control acts as a target value for the inflow control, which then regulates the mass flow to be metered. In this way, individual streams of components or of several components in a single stream can be dosed into the reactor. The feed flow can 3.5 to 0.01 times the removal, based on mass. Preferably, the feed flow is 2.5 times to 0.1 times of the removal, more preferably 1.75 times to 0.25 times.

This has the advantage that available volume in the reactor chamber is used for the (trans)esterification reaction, which increases the space-time yield. Preferably, further alcohol, amino alcohol or amine starting material is added to the maximal reactor filling level in order to use the maximal volume of the reactor for the (trans)esterification reaction and in order to even more increase the space-time yield. This has the further advantage that an optimal heat transfer from the outer wall into the reaction wall into the reaction medium is achieved as a maximum utilized heat transfer area is provided. Further preferably, the reactor filling level is maintained constant during the (trans)esterification process.

When performing the method according to the invention, it is a further preferred option that the concentration of the side product in the column head is kept constant. This has the advantage that the separation of compounds in the reactor system is optimal and thus the loss of e.g. MMA from the system is decreased.

With reference to figure 1, it is illustrated that the amount of reflux (7) to the column head (3) may be controlled via the fill level in the reflux tank (6). If more distillate accumulates from the condenser system (5), the amount of reflux will increase if the fill level in the reflux tank (6) rises. If no distillate is withdrawn from the system via the distillate transfer line (8), all condensate obtained from the condenser system (5) is fed to column via a reflux tank (6) and a reflux line (7). In the process step of transesterification, it is preferred that a controller ensures that there is always a transfer of distillate via a transfer line (8) to a receiver vessel (9). This is done from the calculation of the reflux ratio (v = reflux stream in the reflux line (7) / distillate transfer in line (8)). The distillate transfer stream (8) is adjusted so that the reflux ratio (v) is always in a range of maximum 15 and minimum 0.33. The transfer stream (8) can be freely selected between the specified limits, which are defined by the reflux ratio (v). However, this is preferably set in such a way that a constant column top concentration of methanol in the column head (3) can be maintained. The concentration at the top of the column (3) is determined using the flow meter (10) in stream (7) or (8).

The concentration should be kept between 78% by mole (MeOH) and 62% by mole (MeOH), preferably 74% by mole (MeOH) and 68% by mole (MeOH)). If the concentration of methanol in the column head (3) increases, the transfer stream (8) increases; if the concentration of methanol in the column head (3) decreases the transfer stream (8) decreases.

The side product concentration can be determined via a measurement, which can be performed online. For example, the refractive index and/or density can be measured. The column head take-off can be adjusted via the concentration measurement so that the column head concentration remains constant over the course of the reaction. The side product, which is methanol, when MMA is used as a starting material, accumulates during the reaction, and is continuously separated via distillation through the connected column. If the concentration of methanol in the column head is kept constant within a determined range of 78 to 62 % by mole, preferably 74 to 68 % by mole, then the separation from the reactor and thus the loss of MMA from the system is optimal. However, a minimal amount of take-off is always kept keeping the reaction process in progress even with only low conversions at the end of the reaction.

According to the invention, the concentration measured at the top of the column may act on the amount withdrawn via a calculation and regulation. The return flow to the column may be controlled by the filling level of the distillate collection tank. If nothing is withdrawn from the system, the resulting distillate is completely returned to the column as reflux. The calculation in the process control system may ensure that a maximum and minimum reflux ratio is maintained, regardless of what the concentration measurement at the top of the column indicates.

The calculation scheme may be as follows:
a) measurement of the concentration (top of the column),
b) control of the concentration (to the setpoint),
c) conversion of the controller output to a draw-off volume flow,
d) limitation of the draw-off volume flow, including the maximum and minimum reflux ratio (range 15 max. 0.33 min.), and
e) set point of the fume hood controller.

It is a further preferred option of the method of the invention that the energy input into the reactor chamber is controlled via the total amount of distillate produced.

According to the invention, the energy input into the reactor (1) is realized via the heating system (12, 12a) of the reactor. The system can be heated e.g. via steam, thermal oil system, electrically, etc.

The required heat output (energy input into the reactor) is typically regulated in such a way that certain parameters (total distillate occurring in the condenser system (5), pressure drop across column (2) and reactor temperature) are not exceeded during the process. The main control variable for the energy input is typically the amount of the distillate stream occurring in the condenser (5) or the reflux tank (6). This should be kept constant throughout the reaction. The amount of distillate produced should be chosen such that for the column (2) an F factor in the range of 0.5 to 3.0 Pa^(1/2), preferably in the range of 1.0 to 2.0 Pa^(1/2) is achieved. If only the pressure loss in the column rises above a preset value in the range of 10 to 4 mbar/m packing, preferably in the range of 6.5 to 4 mbar/m packing, the energy input via the heating and/or cooling system (12, 12a) should be reduced to such an extent that this value can be maintained. If the temperature of the reaction chamber or the wall of the reaction chamber rises above a preset value of 150°C to 185°C, preferably 160°C to 170°C, the energy input should be adjusted so that this value can be maintained. This strategy for the energy input can ensure that the column (2) and the reactor (1) are not loaded beyond their system limit, but that the system is always at its upper operating limit and is therefore optimally utilized.

The energy input into the reactor can be controlled via the total amount of distillate produced. The measured distillate quantity can be corrected by possible level fluctuations of the condensers. The mass flow vapors flowing from the column into the condensers can be kept constant. However, the distillate flowing from the column into the condenser can be limited by the two parameters. If the wall temperature at the location of the heat transfer is above a critical value, the amount of energy flowing into the reactor can be regulated downwards in order not to exceed this critical temperature. The critical temperature is typically in the range of 150°C to 185°C, preferably 160°C to 170°C. This is important as otherwise the operating-time of the reactor is reduced, and the polymerization tendency of the product is reduced. The pressure difference across the column including the vapor line can also be measured continuously. If the pressure loss rises above a critical value, which can be defined as or is in the range of 10 mbar/m to 4 mbar/m for structured packing, preferably in the range of 6.5 mbar/m to 4 mbar/m for structured packing, the amount of energy entering the reactor is also regulated downward. This prevents foam from entering the column, which leads to a drastic reduction in operating time and performance. Due to these two situation-dependent interventions, however, a high performance, energy input, can still be operated over the complete reaction time, since if a limitation through pressure difference or wall temperature were to occur in certain process sections, the system intervenes in a self-securing manner. I.e., only in small process sections, or process time phases, intervention reducing the performance are made. the rest of the time the system can be operated at full capacity can be run at full power. This as well leads to an increase of the space-time yield.

In an even further preferred option of the method of the invention the end point of the (trans)esterification reaction is determined via the concentration of the side product in the column head, the temperature in the column head and the reactor temperature.

If the reaction is completed, this point could be reliably detected to be able to initiate the next process step, a vacuum phase, or a distillation phase. The vacuum or the distillation phase starts if the concentration of methanol in the column head (3) reaches 5% by mole, preferably 3% by mole, and more preferably 2% by mole.

As mentioned, the end point of the (trans)esterification reaction can be determined through the combination of values regarding the column head concentration and/or temperature and reactor temperature. This has the advantage that the risk of polymerization can be limited and an increase of the concentration of secondary components (based on side reactions) may be prevented.

In the conventional mode of operation, only the reactor temperature was used to detect the end point.

According to a preferred option of the inventive method the removal of the side product is stopped when an end point of the vacuum phase is reached.

In the alternative, the end point of the vacuum phase in case of low-boiling esters is determined from the pressure and the temperature profile of the column, and the removal of the side product is stopped when the end point of the vacuum phase is reached.

The end of the process in the production of low-boiling esters can be identified by identifying a characteristic temperature profile. Temperature sensors can be installed at different heights in the column (2). These can be located (a) at the top of the column, (b) after the 1st 1/3 of the column packing, (c) after the 2nd 1/3 of the column packing, and (d) in the column bottom. The end of the process can be defined in such a way that sensors (c), (b), and (d) must have nearly the same temperature. If this is the case, the temperature can be recorded in (a). The recorded temperature in (a) then can be subtracted from the temperature (d). If the temperature (a) now increases by 1/3 of the calculated value, the end of the process has been reached.

As the reaction end point detection, the detection of the end of the whole process, which can also be the end of the vacuum phase, is important. This has the advantage that when the vacuum phase is finished a final purity can be guaranteed. Furthermore, detecting the end point of the reaction has the further advantage that the vacuum phase is not lasting too long, and a risk of polymerization is decreased. The needs-based recognition increases overall the space-time yield.

Preferably, the (trans)esterification reaction is carried out in the presence of a polymerization inhibitor. This can be entered to the system as solid and/or fed as solution of one or more components of the reaction mixture anywhere in the system.

This has the advantage that polymerization is inhibited, and the space-time yield is increased. Furthermore, the product can be purer.

As an inhibitor, p-phenylenediamines, phenothiazine and hydroxylamines like N,N-bis(2-hydroxypropyl)hydroxylamine (HPHA) and N,N-diethylhydroxylamin (DEHA) can be used.

Most preferred inhibitors or stabilizers are hydroquinone (HQ), its methyl ether HQME and 2,2,6,6-tetramethylpiperidinyloxyl (TEMPO), or its derivatives like 4-hydroxy-2,2,6,6-tetramethylpiperidinyloxyl (hydroxy-TEMPOL) and any derivative of this functionalizing the hydroxy group like methacrylic ester of 4-hydroxy-2,2,6,6-tetramethylpiperidinyloxyl.

The polymerization inhibitor hydroquinone methyl ether (HQME) can be present in the range of 1 ppm to 2000 ppm, preferably 10 to 1000 ppm, more preferred 10 to 500 ppm, and most preferred 50 to 100 ppm. The polymerization inhibitor 2,2,6,6-tetramethylpiperidinyloxyl (TEMPO) or any of its derivatives can be present in the range of 0.1 ppm to 1000 ppm, more preferred 1 ppm to 500 ppm, and most preferred 10 ppm to 100 ppm, with ppm in weight.

Preferably the (trans)esterification reaction is carried out with introduction of oxygen into the reaction mixture.

This has the advantage that the reaction is stabilized, and the reaction speed is accelerated.

Beside oxygen also air can be introduced into the reaction to speed up the reaction process. The oxygen may be introduced continuously or discontinuously, preferably the oxygen is introduced continuously.

In the method according to the invention, the (meth)acrylate starting material may comprise methyl (meth)acrylate, and the side product may comprise methanol, or the (meth)acrylate starting material may comprise ethyl (meth)acrylate, and the side product may comprise ethanol, or the (meth)acrylate starting material may comprise n-butyl (meth)acrylate and the side product may comprise butanol, or the (meth)acrylate starting material may comprise (meth)acrylic acid, and the side product may comprise water, preferably the (meth)acrylate starting material comprises methyl (meth)acrylate, and the side product comprises methanol.

### Sequence of the overall process:

The overall process of the (trans)esterification process can be characterized by a particular sequence of sub steps. These are determined by the product to be manufactured and may vary depending on the product.

Basically, the following steps can be run through in the (trans)esterification process:
- filling and feeding of the reactants in the reactor,
- addition of stabilizer,
- optionally dewatering of the mixture,
- addition of catalyst,
- heating of the reactor contents,
- separation of byproduct (e.g. methanol) during reaction to the target product under adjusted pressure and temperature,
- optional addition of reactants during the reaction phase,
- further lowering of the pressure for separation of the excess starting material and transfer it to the feedstock for the next reaction batch,
- detection of the end point of the process,
- adjustment of pressure and temperature for the next process steps.

Further advantages, details and features of the invention will be apparent below from the examples explained.

### Examples

### Example 1: Preparation of EHMA (2-ethylhexyl methacrylate)

2-Ethylhexyl methacrylate (EHMA) was produced in the reaction system shown in Fig. 1. 2.26 kg of MMA (methyl methacrylate) were placed in the stirred reactor (1) and, after adding 2.62 kg of 2-ethylhexanol, 8 g of catalyst (ethylhexyl titanate) and stabilizer (4-hydroxy-TEMPO, hydroquinone monomethyl ether) were heated to 100°C. The reactor (1) was continuously flushed with air. The pressure maintenance was set to 1.05 bar. Once the heating was complete and the temperature of the stirred reactor (1) was above 100°C, the energy supply was regulated in such a way that a constant amount of distillate accumulated at the top of the column condenser (5).

All of the condensate obtained was returned from (6) via (7) to the top of the column (3). Once the temperature at the top of the column (3) fell below 70 C, the low boilers (methanol) began to be separated off from the system via line (8) into receiver vessel (9). The concentration of the low boilers in the discharge stream (8) was determined by the mass flow meter (10). The draw-off stream was regulated in such a way that the low boiler concentration (methanol) was between 68% by mole and 72% by mole. Equivalent to withdrawal, MMA was added to the reactor via the feed line (13) so that the level in the reactor (1) was maintained. The amount added over the reaction time was 7.3 kg. The temperature in the reactor (1) was kept constant at 125°C via the pressure in the reaction system.

If the pressure in the reaction system reached 600 mbar, this was maintained at 600 mbar, independently of the temperature in the reactor (1).

If the temperature at the top of the column, measured in (3), reached a value of 85°C, the receiver vessel (9) was emptied via the distillate discharge line (15). The resulting distillate from (8) was then collected in (9). This was then used for the next production in (1).

If the heating steam temperature in (12) exceeded 155°C or if the differential pressure in the column (2) increased to more than 40 mbar, the energy input into the heating system (2) was throttled.

The ratio measured across (10), between (7) and (8), was kept in the range between 15 and 0.33 by adjusting the draw stream (8).

If the reactor (1) reached a temperature of 132°C, the system pressure was further reduced to the final pressure of less than 20 mbar. The temperature in the reactor (1) was kept constant at 110°C via the energy input in (2). The pressure reduction in the reaction system was carried out in such a way that the total amount of distillate (total from the outlet (8) and reflux (7)) remained constant. All distillate obtained at the top of the column condenser (5) was then transferred to the receiver vessel (9).

If the MMA content in the reactor (1) fell below 1% by mole, it was cooled to less than 60°C via (2) and then emptied via (14) for further processing.

## Claims

1. Method for preparing an alkyl (meth)acrylate product by a (trans)esterification reaction of a reaction mixture in a reactor system, the reactor system comprising a heating and/or cooling system (12, 12a), a reaction chamber (1) comprising the reaction mixture, a column (2) with a column head (3), a vapor transfer line (4), a condenser system (5), a reflux tank (6), a reflux line (7), a distillate transfer line (8), and a receiver vessel (9),
wherein the reaction mixture comprises a (meth)acrylate starting material and a first alcohol which are converted by the (trans)esterification reaction at a reaction temperature and a given pressure in the reaction chamber in the presence of a catalyst and other additives into the alkyl (meth)acrylate product and a side product,
wherein over a predominant amount of time during the (trans)esterification reaction at least a portion of the side product is continuously or incrementally removed by distillate take off, and
wherein over a predominant amount of time during the (trans)esterification reaction the given pressure is constantly or incrementally adjusted to keep the reaction temperature below a predetermined upper limit.

2. Method according to claim 1, wherein the constant adjustment of the given pressure is a constant linear decrease of the given pressure to a target value.

3. Method according to one or more of the preceding claims, wherein the side product is removed in form of a mixture of the side product and the (meth)acrylate starting material.

4. Method according to one or more of the preceding claims, wherein a filling level of the reaction chamber is kept constant by compensating at least a portion of the converted (meth)acrylate starting material and/or of the first alcohol by adding a further amount of the (meth)acrylate starting material and/or of the first alcohol to the reaction mixture in the reaction chamber, preferably via a feed line to the reaction chamber or to the column.

5. Method according to one or more of the preceding claims, wherein the concentration of the side product in the column head is kept constant.

6. Method according to one or more of the preceding claims, wherein the energy input into the reactor chamber is controlled via the total amount of distillate produced.

7. Method according to one or more of the preceding claims, wherein the end point of the (trans)esterification reaction is determined via the concentration of the side product in the column head and/or the reactor temperature and/or the temperature at the top of the column.

8. Method according to one or more of claims 1 to 7, wherein the removal of the side product is stopped when an end point of the vacuum phase is reached, wherein in case of high-boiling esters the end point of the vacuum phase is determined by a collapse of the distillate volume.

9. Method according to one or more of claims 1 to 7, wherein the removal of the side product is stopped when an end point of the vacuum phase is reached, wherein in case of low-boiling esters the end point of the vacuum phase is determined from the pressure and the temperature profile of the column.

10. Method according to one or more of the preceding claims, wherein the (trans)esterification reaction is carried out in the presence of a polymerization inhibitor.

11. Method according to one or more of the preceding claims, wherein the (trans)esterification reaction is carried out with introduction of oxygen into the reaction mixture.
